# EUROPEAN PATENT APPLICATION

(11) **EP 2 997 887 A1**
(43) Date of publication of application: **23.03.2016**
(21) Application number: 15185237.3
(22) Date of filing: 15.09.2015
(51) Int. Cl.: A61B 5/00, A61B 5/044, A61B 5/11, A61B 5/16, G06F 19/00

(54) **VIRTUAL ENVIRONMENT UTILIZING NEUROPHYSIOLOGICAL ANALYSIS AND ANALYTICAL METHOD USED THEREIN**

(30) Priority: 16.09.2014 FI 20145809
(71) Applicant: Satavision OY, 90240 Oulu (FI); Exakti Intelligence Oy, 00170 Helsinki (FI)
(72) Inventor: Sorvisto, Mika, 90440 Kempele (FI); Lindqvist, Tony, 90240 Oulu (FI); Kivikangas, Markku, 00170 Helsinki (FI)
(74) Representative: Berggren Oy Ab

(57) **Abstract**

The 3D virtual environment (40) of the invention comprises advantageously the main parts of a traditional CAVE space. The 3D virtual environment has projection means (43) for projecting images of the object under review (3a) on the surface of at least one display element of the 3 virtual environment. The 3D virtual environment further comprises at least one computer for generating the images of the virtual environment and for recording the EEG measurement data produced by the brain function the object under review generates in the viewer's brain from EEG signals generated by the EEG hat (4) the viewer wears. A brain response generated by the object under review (3a) is edited from the recorded EEG data by neuro analytical methods, the emotional and/or cognitive state experienced by the viewer (10) at the moment of the test being identifiable from the brain response.

## Description

The object of the invention is a three-dimensional virtual environment, on the walls and ceiling of which images of an object under review are projected, determined by the place and position of the viewer. In this virtual environment the viewer's reactions are measured by neurophysiological methods, for example by electroencephalogram (EEG). In EEG electrodes are attached to the person's scalp for measuring electric potentials caused by brain activity in different parts of the brain.

### State of the art

In a test situation, the reaction of a test subject can be measured with different measuring arrangement measuring physiological signals. Heartbeat can be monitored with electrocardiogram (ECG), changes in galvanic skin response (GSR) can be measured with resistance measurements. The electric activity of a test subject's face muscles can be measured with electromyography (EMG). Optical systems can be used for filming the test subject's micro expressions, which are difficult to hide. Micro expressions are momentary, very short involuntary facial expressions lasting only for 1/25 - 1/15 seconds. Micro expressions are usually born in stressful situations. Micro expressions occur when a person consciously tries to hide all signals of what their true feeling sensation is.

One way to measure the effectiveness of advertising is disclosed in the application publication US 2013/0185144. In the measuring arrangement of the publication the effectiveness of advertising is concluded on the basis of delta, theta, alpha, beta and gamma EEG frequencies measured from the test subject's brain. The evaluation is based on the differences between two measured EEG frequencies.

There are also 3D-based virtual environments, which are generally called by the name CAVE (Computer Assisted Virtual Environment). CAVE® is a registered trademark of the Illinois University, and it refers to the original virtual space according to the Illinois University. However, the word CAVE is also used as a general attribute when speaking about spaces similar to the virtual space developed by the Illinois University.

A conventional CAVE system (or later CAVE space) is an installation, with which it is possible to create a realistic 3D virtual environment around the user. The CAVE space can resemble a small room, the walls of which consist of one or several projection screens. The image of the projection screens is usually formed by projectors as front or back projection. Also different display panels can be used for forming the projection screens. A stereoscopic image of the virtual environment is formed on the projection screens from the user's perspective so that the 3D virtual environment and its 3D objects can be shown to the viewer in their natural size. The perspective of the views can be changed by means of the viewer's positioning system, if the user is moving in the 3D virtual environment. In this case the user can, for example, see under an object in the virtual environment when crouching down.

The technical implementation of the CAVE space varies depending, for example, on the size and characteristics of the system. The system can include the apparatus frame, display surfaces, projectors, computer system with software, positioning system, 3D glasses as well as location and position identification apparatus for indicating location and position.

Display surfaces of the CAVE space are usually realized with projectors, because using them it is easy to make big uniform projection screens. Display surfaces made by means of projectors can be realized either as front or back projection. In front projection the image formed by the projector is reflected on a non-transparent surface from the viewing side. In back projection the image formed by the projector is reflected on a semi-transparent surface from the other side of the viewing surface. Which projection technique is used, usually depends on how many display surfaces the CAVE space is built. Systems with several walls are conventionally made with back projection. In this case the placing of projectors and their users do not create shadows on the projection screens of the CAVE space. In the building of a CAVE space the intention is to avoid seams or interruptions in the image area, because they interfere with the observation of the depth of a stereoscopic image.

In addition to the projection technique, it is known for one skilled in the art that it is possible to make the images shown in the CAVE space using also other known virtual display techniques.

The size, direction and number of display surfaces in the CAVE space may vary, but generally they cover as much of the user's field of vision as possible in order to maintain the illusion of a surrounding virtual environment.

By means of stereoscopy, a dimensional effect is achieved for the images on the display surfaces of the CAVE space, when needed. This is usually made by copying the binocular depth hints of the depth perception. In this case two-dimensional images slightly different from each other are shown to the viewer's left and right eye so that the viewer's brain interprets the images as one three-dimensional image of the object under review.

In CAVE spaces it is possible to achieve stereoscopy, for example, with 3D glasses, by means of which it can be determined which kind of images (deviating from each other) the left and right eye see. Such stereoscopy can be realized either with active or passive technology. In active technology the 3D glasses contain electronics, which darken the lenses of the 3D glasses alternately so that the left and right eye see through the lenses in turns. The alternatingly occurring darkening of the lenses occurs from several tens of times to several hundreds of times in a second. When a display panel or a projected image changing in the same time with the alternation of the glasses is connected to the operation of the active glasses, different images can be shown to the left and right eye.

In passive technology the 3D glasses do not contain electronics. The lenses of the 3D glasses of the passive technology function as a filter for beams of light only letting through light with a certain characteristic. For example, the lenses can filter different colours of light (spectrum) or the direction or vibration of the light's wave motion (polarization). When the filtering for the left and right lenses of the 3D glasses used differ from each other, it is possible to show a different view to the viewer's left and right eye by filtering the beams of light arriving from the image source with the lenses of the 3D glasses.

Generally polarized light is used in passive CAVE spaces. Such an arrangement requires two projectors for each projection screen. The one projector shows the image intended for the right eye and the second one the image intended for the left eye. There is a polarizing filter in front of the lens of both projectors. When the reflected image is viewed with passive 3D glasses with polarizing filters corresponding to the projectors, the viewer's left and right eye see two images polarized in a different way. In order to operate the polarization technique requires a display surface maintaining the polarization plane of the light.

In the future the stereoscopic image production in CAVE spaces can be realized using several different display techniques, such as, for example, an autostereoscopic display.

The stereoscopic image to be shown on the display surfaces is produced by an appropriate computer system. The computer system calculates and has drawn a view of the 3D virtual environment on each display surface seen from the viewer's perspective. The computer system can consist of one or several computers. In a one-computer system the image signals of all projection screens are produced from one computer with several different image outputs. The system is simple to use and its operation is more reliable compared with a system with several computers.

In a system with several computers the computers are networked with each other as a cluster. In the cluster one of the computers functions as the master machine, which guides the other computers via the net. The image outputs of the computers are synchronized with each other so that the stereoscopic image would remain on all projection screens at the same phase. The cluster's advantage is the combined computing power, but its disadvantage is the complexity and expensiveness of the system.

A separate positioning system is used in the CAVE spaces for the determination of the user's location and viewing direction. On the basis of the viewer's location and direction, the computer in the CAVE space calculates the view of the projection screens of the 3D virtual environment seen from the viewer's perspective. As the viewer moves the CAVE space adapts the perspective of the views to correspond to the viewer's new location. The positioning technique can be optical so that one or several cameras are attached to the frame structures of the CAVE space to film the viewer. On the basis of the views of the cameras, the computer calculates the location and direction of the viewer. When using 3D glasses, they can be provided with markers, by means of which the location and direction of the viewer's eyes can be calculated more accurately.

3D presentations can also be created on a two-dimensional (2D) display. One example of a three-dimensional display system realized on a 2D display is a 3D design program. In the 3D design program the program application guiding the drawing of the image on the display apparatus draws an image of the object under design, which the viewer perceives as three-dimensional. If the object is still turned on the 2D display, the 3D impression the viewer gets from the shown object is intensified.

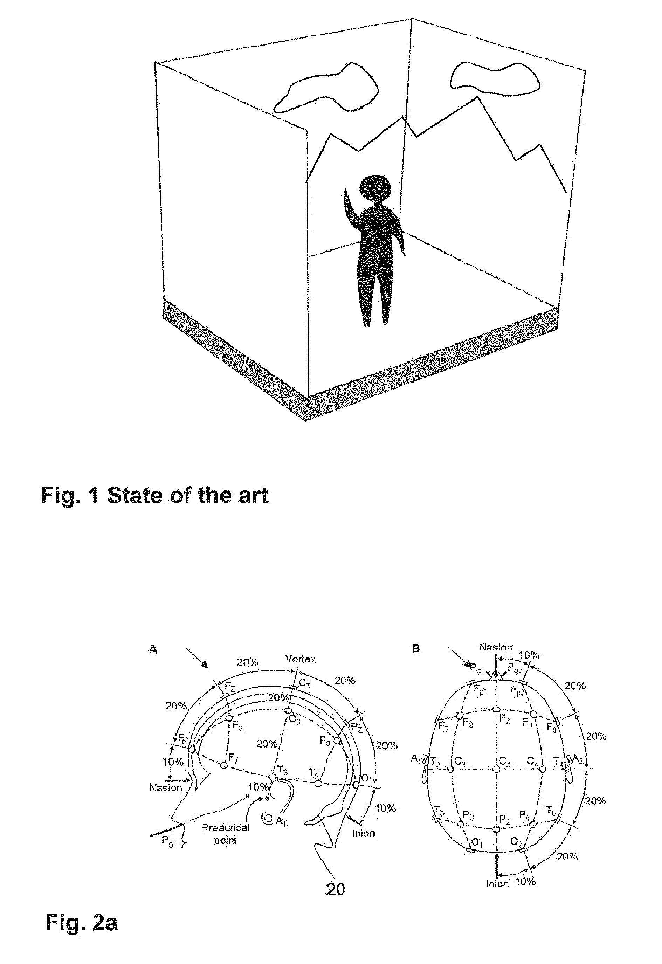

**Figure 1** illustrates an example of a conventional CAVE space (figure Illinois University). In the figure the viewer is in the CAVE space, in which stereoscopic image is projected on three walls and the floor surface.

The 2D or 3D display solutions described above can be used, for example, in the presentation of things, products or places. Sensations or preferences of the object's viewer of what they've seen are examined afterwards with oral queries or different questionnaires. In the disclosed systems the viewer's actual experiences of what they've heard or seen can be recorded only some time after the actual moment of experience. Then the different things or occurrences not linked with the actual experience can revise the feedback given by the viewer and thus the feedback is not realistic for all parts and can thus distort the final result of the survey.

### Object of the invention

It is an object of the invention to introduce a new 3D virtual environment, in which the reactions of the viewer in the 3D environment to the stimuli generated by the object under scrutiny are measured and indicated real-time and without problems caused by queries or interviews.

The objects of the invention are achieved with an apparatus arrangement, in which the viewer's brain function is measured in 3D virtual environment by using, for example, EEG. The user is presented with a visual stimulus, for example a product, in the 3D virtual environment, the activity generated by which in the brain is measured by means of EEG electrodes placed in the user's head. This is possible when the place of the response in the brain, its frequency and intensity are known. The result reflects both the viewer's conscious and subconscious reactions to the visual stimulus.

An advantage of the 3D virtual environment of the invention is that the viewer's real reactions to the visual stimulus can be indicated and recorded.

It is further an advantage of the invention that the subconscious reactions the stimulus caused to the viewer can also be indicated.

It is further an advantage of the invention that the viewer's visual target in the virtual environment can be determined by means of the viewer's location and the position of their eyes.

It is further an advantage of the invention that the viewer's reactions to the shown stimulus can be measured real-time.

It is further an advantage of the invention that the viewer's reactions can be measured in a desired timeframe so that it is possible to indicate the viewer's true first reactions, when also the showing time for the stimulus is known.

It is a further advantage of the invention that the time of occurrence for the viewer's reaction can be accurately determined in the system. Then it is always known which measuring response has been achieved with which visual stimulus.

It is further an advantage of the invention that the reactions caused by the stimulus in the viewer can be indicated, irrespective of the viewer's nationality or language.

It is further an advantage of the invention that it is not necessary to concretely build the products or environments to be tested so that savings in time and costs can be achieved.

It is further an advantage of the invention that the feedback by the real user groups of products or premises can be made to support the design process and results of several options can be recorded also for further use.

It is characteristic of the 3D virtual environment of the invention that it comprises means for determining the brain response generated in the viewer's brain by the object shown to the viewer in the 3D environment.

It is characteristic of the method of the invention for determining the mood the image presenting the object under review creates in the viewer, in which method the viewer is shown a three-dimensional image of the object under review in 3D environment, that in the method it is further determined the brain response the object under review generates in the viewer's brain.

Some advantageous embodiments of the invention are shown in the dependent patent claims.

The basic idea of the invention is the following: The 3D virtual environment of the invention comprises advantageously the main parts of a traditional CAVE space. In the 3D virtual environment there are projection means for projecting the images of the object on the inner surface of at least one wall element of the 3D virtual environment. The 3D virtual environment of the invention further comprises at least one computer for generating the images needed in the virtual environment and for feeding them to the projection means and for saving and analysing the EEG measurement data generated in the measuring transaction. In the 3D virtual environment of the invention the positioning of the viewer is based, for example, on a depth camera filming the viewer, of the image of which the computer advantageously belonging to the 3D virtual environment concludes the viewer's location and position by using a shape recognition software. The positioning system used does not need markers attached to the viewer to operate.

In an embodiment of the invention the 3D virtual environment can also include a measuring apparatus registering the position and movements of the viewer's eyes. In this case the viewer's visual target in the 3D virtual environment can be determined.

In the 3D virtual environment of the invention stereoscopy is advantageously achieved by active 3D glasses in the viewer's possession, the stereoscopy darkening alternately the lenses of the 3D glasses so that the user's left and right eye see alternately the images generated in the 3D virtual environment through the lenses of the 3D glasses. The projectors of the 3D virtual environment of the invention feed two images differing slightly from each other advantageously on the frequency of 120 Hz. In this case the alternate darkening of the lenses for the left and right eye of the 3D glasses advantageously occurs on the frequency of 60 Hz. The viewer's brain then edits a three-dimensional visual experience from the information arriving to the left and right eye.

Electrodes are placed to the head of a person coming to the 3D virtual environment of the invention, the electrodes measuring the EEG signals generated in the viewer of the object shown in the 3D virtual environment. The said EEG measuring apparatus can also advantageously comprise a wireless data transmission apparatus, with which the real-time EEG measurement data is transferred to the computer maintaining the 3D virtual environment to be recorded for further analysis.

The computer of the 3D virtual environment of the invention comprises a program application, which processes the received EEG signals so that the place, frequency and intensity of the brain function the object generates in the viewer can be determined. In researches it has been found out which sectors of the brain are activated in different cognitive or emotional states. When it has been discovered which parts of the brain have been activated by the stimulus and how, the said program application can be used to draw conclusions from this measurement data as to which are the viewer's real reactions to the stimulus.

The invention is next explained in closer detail, referring to the enclosed drawings in which
Figure 1 illustrates a known CAVE space;
Figure 2a illustrates in an exemplary manner an EEG electrode arrangement used in the 3D virtual environment of the invention;
Figure 2b illustrates in an exemplary manner an EEG measuring arrangement used in the 3D virtual environment of the invention;
Figures 3a-3 illustrate examples of different ways to present the activation of brain function;
Figure 4 illustrates in an exemplary manner the functional main parts of the 3D virtual environment of the invention; and
Figure 5 illustrates as an exemplary flowchart a method for analysing the reactions of the object used in the 3D virtual environment of the invention.

The embodiments in the following specification are only exemplary, and one skilled in the art can carry out the basic idea of the invention also in some other way than the one disclosed in the specification. Although reference can be made to an embodiment of embodiments in several places in the specification, this does not mean that the reference would only be aimed at one described embodiment, or that the described feature would only be feasible in one described embodiment. Individual features of two or several embodiments can be combined and thus achieve new embodiments of the invention.

The 3D virtual environment of the invention makes use of an EEG measuring technique used in the neurophysiological analysis. In this case the test subject in the 3D virtual environment wears on their head a hat, in which the EEG electrodes are either preinstalled or to which they will be installed individually for each user. The EEG electrodes in the EEG hat are used for measuring the functional state of the brain in real time. The activity generated by the stimulus or stimuli in the nerve cells of the brain generates an electric signal, which can be indicated through the bones in the skull of the tested person with the sensors in the EEG hat. The place of activity in the brain generated by the shown stimulus can be processed from the EEG measurement data, and advantageously, its frequency and the intensity of activity generated by the stimulus. During the test the EEG data monitoring the electric function of the brain is advantageously recorded in the computer so that the connection between the shown stimulus and the response following it can be examined.

**Figure 2a** discloses an example of an advantageous way to position the EEG electrodes used in EEG measurements around the head by means of an exemplary EEG hat. In the example in Figure 2a the hat has nineteen EEG electrodes, which in Figure 2a are marked with the references Cₙ, Fₙ, Pₙ and Tₙ. The EEG sensors can be either of a wet or dry type. The illustrated EEG sensors cover the upper section of the skull 20 of the person under examination from the forehead above the eyes and above the ears all the way to the neck. It is obvious for one skilled in the art that the EEG sensors are not only restricted to the nineteen sensors described in Figure 2a but there can be 1 - 512 of them.

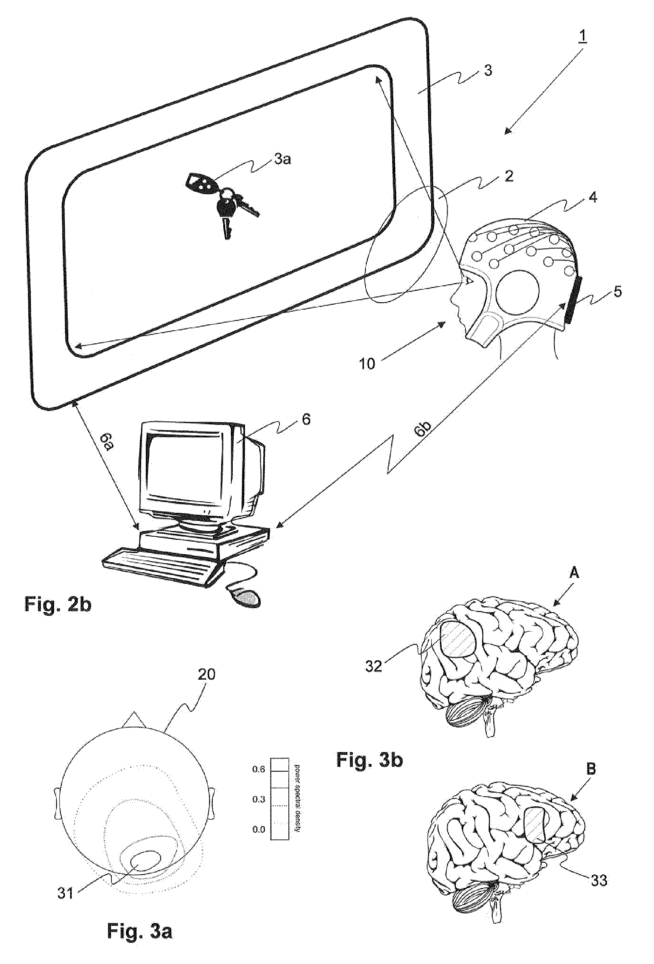

Figure 2b illustrates in an exemplary manner the apparatus arrangement 1 used in the neurophysiological analysis. In the example in Figure 2b the reactions of the viewer 10 either to a 2D or 3D image or images or video on the display device 3 are measured by means of the EEG hat 4. The electrodes of the EEG hat 4 are connected to the amplifier 5 connected to the EEG hat 4. From the amplifier 5 there is either a wired or wireless connection 6b to the computer 6 controlling the test situation. The computer 6 is also connected to the display device 3 used in the neurophysiological analysis with the connection 6a. Through the connection 6a the computer generates individual images or videos on the display of the display device 3, the reactions of which to the viewer 10 one wishes to examine. In the example in Figure 2b the stimulus caused by the object 3a under review in the viewer's 10 field of vision 2 can be located by the EEG measurement, likewise the strength of the response caused by the stimulus seen by the viewer.

The EEG signal to be received from the EEG electrodes of the EEG hat 4 is analogue, so it has to be converted into digital in order to be able to process it in the computer 6. After the conversion into digital the EEG data can be filtered in a desired way using an appropriate digital filter.

In the neurophysiological analysis it is common that the same stimulus is repeated several times so that it is possible to filter disturbance signals caused by external effects out from the measurement data. In this case one examination session can last from, for example, 10 minutes to as long as 45 minutes. Examples of above-mentioned disturbance signals are an alternating-current hum of 50 Hz, movement of the eyes and non-desired voices during the measurement. In addition, it is the intention in the neurophysiological analysis to randomize the viewer's situation so that, for example, the presentation order of examined images is randomized in order to prevent the viewer from getting used to the shown stimulus. It is possible to average the results from different measurements to improve the measurement results.

In the example in Figure 2b a visual stimulus has been used as the stimulus for the neurophysiological analysis. It is obvious for one skilled in the art that the described neurophysiological analysis arrangement can be used for measuring the brain response also for other kinds of stimuli. Examples are hearing stimulus, taste stimulus, smell stimulus and sense stimulus (e.g. pain stimulus). In the 3D virtual environment of the invention it is thus possible to also measure brain responses generated by other senses in addition to the visual stimulus.

In the EEG measurements the intensities of brainwaves in different frequency areas can be found out: delta (0.5 - 4 Hz), theta (5 - 7 Hz), low alpha (8 - 10 Hz), high alpha (11 - 13 Hz), beta (14 - 30 Hz) and gamma (36 - 44 Hz). Changes in the amplitudes of brainwaves caused by brain activity can be measured with an accuracy of milliseconds and µV.

The same brainwave frequency and intensity of amplitude in different parts of the brain means a different brain response, which has been generated by a certain stimulus.

**Figure 3a** illustrates an example of an activity map edited from EEG data. In the example in the figure the stimulus shown to the viewer has been activated in the brain section 31 at the end of the brain facing the back of the head. Such a response is usual for a visual stimulus.

The partial figures **A** and **B** of **Figure 3b** illustrate the asymmetrical brain responses 32 and 33, which can be seen in different sides of the brain and which illustrate the change in the test subject's level of motivation caused by the stimulus.

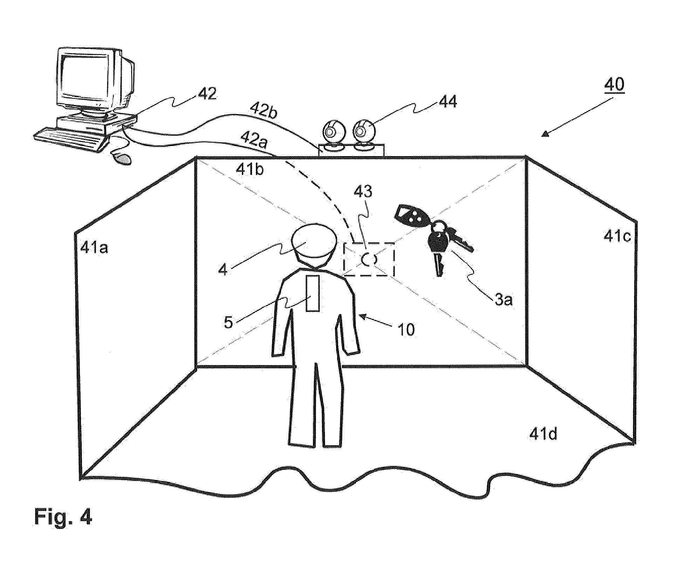

**Figure 4** illustrates an example of the 3D virtual environment 40 of the invention. The 3D virtual environment comprises three display surfaces 41 a, 41 b and 41 c, on each of which it is possible to reflect images in the 3D virtual environment of the invention so that the viewer 10 experiences the view as three-dimensional. In addition also the floor surface 41d and/or ceiling of the 3D virtual environment can be used as a projection screen.

In an advantageous embodiment stereoscopic display technique is used in the projection of images (not shown in Figure 4). In the next explanation of Figure 4 the invention is illustrated by using the above-mentioned stereoscopic technique as an example.

The images shown on the display surfaces or walls 41 a, 41b and 41 c of the 3D virtual environment are controlled by the computer 42, which is connected to the projector 43 with the connection 42a. The computer 42 comprises a processor or processor means, which comprises an arithmetic logic unit, a number of different registers and control circuits. A data recording arrangement, such as a memory unit or memory means is connected to the processor means, to which data or programs readable with a computer can be recorded. The memory means typically contains memory units, which allow both read and write functions (Random Access Memory, RAM) and memory units containing non-volatile memory, from which data can only be read (Read Only Memory, ROM).

The computer 42 also comprises an input or input means for receiving data either of the EEG measurement from the amplifier 5 or from the position identification cameras 44 of the viewer 10. The EEG measurement data or viewer's positioning data received by the input means are transferred to be processed by the processor means of the computer 42.

The computer 42 further comprises output means for transmitting the image information edited by the processor means to the projectors 43 belonging to the 3D virtual environment.

The images of the 3D virtual environment can be projected on walls/display surfaces 41 a, 41 b and 41 c either from the front of the wall or the back of the wall. In the example in Figure 4 there is shown only one exemplary projector 43, which projects stereoscopic images from the back of the wall. In order to provide a perfect three-dimensional effect, the 3D virtual environment advantageously has an own respective projector for each wall. In some 3D virtual environment applications two projectors instead of one have to be reserved for each wall in order to be able to project the different images meant for the left and right eye on the walls.

The 3D virtual environment of the invention can comprise, for example, optical position identification means for determining the viewer's distance and/or position in the 3D virtual environment. As positioning means 44 it is advantageously to use one or several optical cameras, infrared radar, or triangular calculation carried out by base stations of a wireless network. In the example in Figure 4 the optical cameras functioning as optical positioning means 44 are located in the upper part of the middle wall 41 b of the 3D virtual environment, but their place can naturally also be elsewhere in the 3D virtual environment. The positioning cameras 44 are connected to the computer 42 controlling the operation of the 3D virtual environment with the connection 42b.

In an advantageous embodiment of the invention the place of an individual viewer is not determined, but images are projected on the display surfaces of the 3D virtual environment on the basis of known pre-set values. This embodiment is feasible, for example, when there are several persons in the 3D virtual environment at the same time.

The viewer 10 in the 3D virtual environment wears 3D glasses (either active or passive). If the 3D glasses are active glasses, so in this case their operation is synchronized to the shown images by the computer 42, with which the 3D glasses advantageously have a wireless data transmission connection.

In an advantageous embodiment of the invention the 3D glasses can also have sensors following the movement of the viewer's eyes. The visual target of the viewer in the 3D virtual environment can be determined from the measurement data obtained from the sensors.

The viewer 10 in the 3D virtual environment further wears the EEG hat 4, which is connected to the amplifier 5. In addition to amplifier circuits, the amplifier 5 further advantageously comprises an A/D converter, with which analogue EEG measuring signals are converted into digital before transferring the EEG measurement data forward. The amplifier 5 is advantageously connected to the computer 42 controlling the measurement transaction through a wireless data transmission network. It is possible to transfer the EEG measurement data measured via the data transmission connection real-time to the computer 42, in which it will be processed so that the activity in different parts of the brain can be visualized, for example, as a three-dimensional brain image, in which active areas are shown, for example, with certain colours.

From researches of the neurophysiological analysis it is known, which stimulus (positive or negative) activates which part of the brain. Because of this a stimulus produced to the viewer 10 in the 3D virtual environment can be used to find out the viewer's 10 true reaction to the shown stimulus, when the measuring result processed from the EEG measuring result is compared with known neurophysiological analysis results.

In the 3D virtual environment 40 of the invention it is thus possible, for example, to carry out product tests with high reliability. External disturbance factors can be filtered out from the measuring results and the test subject (viewer) is not able to on their own distort the test result in any way. By following the viewer's eye movements it is possible to verify that the obtained measuring signal is a result from the viewing of the object seen by the viewer.

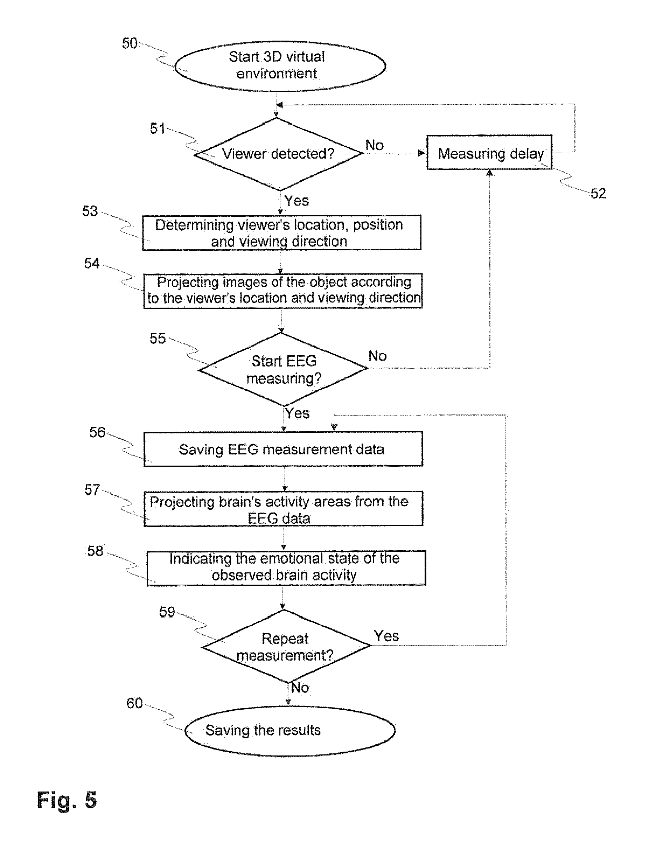

**Figure 5** illustrates as an exemplary flowchart the main steps of an analysis method based on the neurophysiological analysis used in the 3D virtual environment of the invention.

The apparatus assembly formed by the virtual environment is started in step 50. In this case the images seen by the viewer and used in this virtual environment are imaged on at least one wall of the 3D virtual environment with a default viewer distance before transferring to the actual measuring process in step 51.

After the apparatuses of the virtual environment in the 3D virtual environment have been started, also the positioning means of the viewer advantageously transfer to an active state. In this case a decision can be made on the basis of the measurement data obtained from the positioning means in the decision-making step 51, if there is a viewer or viewers in the 3D virtual environment.

If the final result for the decision-making step 51 is the option "No", this means that there is not a single viewer in the 3D virtual environment. The process used in the 3D virtual environment then returns back to step 51 after the measuring delay 52 programmed in the measuring system of the virtual environment, in which the next positioning attempt of a viewer is performed.

The measuring loop through the steps 51 and 52 described above is repeated as long as at least one viewer is observed in the 3D virtual environment in the decision-making step 51. In this case the final result for the decision-making step 51 is "Yes".

If at least one viewer has been indicated in step 51, the place and position of the viewer in the 3D virtual environment can then be determined in step 53, if needed, advantageously using optical position identification means. In the same connection it is possible to follow and determine the viewer's eye movements and positions with separate measuring devices. This measurement can be used for determining the point on the wall of the 3D virtual environment, which the viewer is watching exactly at that moment.

When the viewer's location and position have been defined in step 53, the images on the walls of the 3D virtual environment are then updated to correspond to the determined location and position of the viewer in step 54.

In the neurophysiological measuring process of the invention the viewer advantageously wears an EEG hat on their head. In the decision-making step 55 it is checked if a command to start the EEG measurement has been given. If the final result for the decision-making step 51 is the option "No", the presentation process for the virtual environment will then return back to step 51 through the measuring delay 52.

The measuring loop through the steps 51-56 and the step 52 described above is repeated as long as it is stated in the decision-making step 55 that the viewer's EEG measurement will be activated. In this case the final result for the decision-making step 55 is the option "Yes" and the process continues to step 56.

In step 56 EEG measurement data of the emotional state in the viewer's brain caused by the subject shown in the virtual environment and the related time stamps are recorded. The length of the time for collecting the measurement data can advantageously be selected specifically for each transaction. The obtained measurement data with time stamps are advantageously recorded in the memory of the computer controlling the neurophysiological research. The brain response measured by means of the time stamps linked with the EEG measurement data is always connectable to a certain image of the subject shown to the viewer.

In step 57 the viewer's recorded EEG measurement data are processed in an exploited computer so that the location of the activity generated by the shown stimulus in the viewer's brain, the frequency of the generated activity and the intensity of the generated activity are found out.

In step 58 the measurement data indicating the brain activity of the viewer in the 3D virtual environment are compared with reference data obtained in previous neurophysiological analyses, which contain the statistical connections between different emotional states and measured brain responses.

In the decision-making step 59 it is decided if the amount of EEG measurement data is sufficient. In the decision-making it is taken into account that it must be possible to filter disturbance signals caused by different external effects out from the measurement data by repeating the measurement. In this case one examination session can last from 10 minutes up to 45 minutes. Examples of the above-mentioned disturbance signals are the alternating-current hum of 50 Hz, eye movements or unintentional voices during the measurement. In addition it is always intended in the neurophysiological analysis to randomize the viewer's situation so that, for example, the order of presentation of the images under review are randomized to prevent the test subject from getting used to the shown stimulus. It is possible to average the results of different measuring results in order to improve the measuring result.

If the result for the decision step 59 is the option "Yes", the collection of EEG measurement data is then renewed starting from step 56. The above-described EEG data measuring loop through the steps 56-59 is repeated as long as it can be stated in the decision-making step 59 that the EEG measurement data contains a sufficient amount of data to make a reliable conclusion to define the brain response generated in the viewer by the shown object and further to define the viewer's true emotional state on the basis of this.

If the final result for the decision-making step 59 is the option "No", the measuring results for the viewer in question are recorded for later analysis in the memory of the computer controlling the neurophysiological measurement in step 60.

In a later neurophysiological analysis, which can be conducted after the actual test situation, the true emotional state experienced by the viewer in the test situation is determined by comparing the brain response from the viewer with the reference brain response results obtained in known neurophysiological test measurements.

Some advantageous embodiments of the 3D virtual environment and the analysis method used in it have been described above. The invention is not restricted to the solutions described, but the inventional idea may be applied in numerous ways within the limits set by the patent claims.

## Claims

1. A 3D virtual environment for indicating a reaction caused by an object under review (3a) in a viewer (10), the 3D environment comprising:
- at least one display surface(41 a, 41 b, 41 c, 41 d)
- data processing means (42) for generating three-dimensional images of the object under review (3a), and
- means (43) for presenting a three-dimensional image of the object under review (3a) on at least one display surface (41 a, 41 b, 41 c, 41 d),
**characterized in that** the 3D virtual environment further comprises measuring means (4, 5, 42) for determining a brain response in the viewer's (10) brain generated by the object under review (3a) shown to the viewer (10) in the 3D virtual environment, the brain response being configured to be determined on the basis of a location, frequency and intensity of an EEG signal measured from the viewer's (10) brain, and that an emotional state generated in the viewer (10) by the object under review (3a) has been configured to be determined from the said brain response.

2. The 3D virtual environment according to claim 1, **characterized in that** the 3D virtual environment (40) is a CAVE space comprising at least one display surface (41a, 41b, 41, 41 d).

3. The 3D virtual environment according to claim 1, **characterized in that** the measuring means for determining the viewer's brain response comprises an EEG hat (4) on the viewer's (10) head containing several EEG sensors, an amplifier (5) connected to it and data processing means (42).

4. The 3D virtual environment according to claim 3, **characterized in that** the EEG hat (4) comprises 1 - 512 measuring sensors, which are arranged to cover the upper section of the viewer's (10) skull (20) extending from above the eyes and ears to the neck.

5. The 3D virtual environment according to claim 1 or 2, **characterized in that** he three-dimensional image shown on at least one display surface (41 a, 41 b, 41 c, 41 d) is stereoscopic.

6. The 3D virtual environment according to claim 1, **characterized in that** the 3D virtual environment (40) comprises the viewer's (10) location and positioning identification means (44).

7. The 3D virtual environment according to claim 1 or 6, **characterized in that** the 3D virtual environment (40) further comprises equipment for determining a point of eye alignment of the viewer (10) on the display surface (41 a, 41 b, 41 c, 41d).

8. The 3D virtual environment according to claim 1, 6 or 7, **characterized in that** the said data processing means (42) comprises a program application, which is configured to edit the EEG measurement data obtained from the EEG sensors of the viewer's EEG hat (4) so that it is possible to indicate activations of the brain of the viewer (10) watching the image of the object under review (3a) shown to the viewer (10) in the 3D virtual environment (40) so that an emotional or cognitive state generated to the viewer (10) of the image under review (3a) is configured to be determined from these activations using neuro analytical methods by comparing the neuro analytical brain response generated by the object under review (3a) to known neuro analytical reference brain responses.

9. The 3D virtual environment according to claim 5, **characterized in that** the means (43) for presenting stereoscopic images in the 3D virtual environment (40) comprises a front or back projector for each wall (41 a, 41 b, 41 c) functioning as the display surface for the virtual display environment (40).

10. The 3D virtual environment according to claims 1 or 5, **characterized in that** the projection screens (41a, 41b, 41c, 41d) of the CAVE space (40) are electronic display units, which are configured to present three-dimensional images of the object under review (3a).

11. The 3D virtual environment according to claim 6, **characterized in that** the location and position identification means (44) comprise at least one optical camera, infrared camera or triangulation equipment operating in a wireless network for identifying the viewer's (10) location and position.

12. A method for determining an emotional state generated in a viewer (10) of an image of an object under review (3a), in which method the viewer (10) is shown a three-dimensional image of the object under review (3a) on at least one display surface (41 a, 41 b, 41 c, 41 d) of the 3D virtual environment (40), **characterized in that** in the method a brain response generated by the three-dimensional image of the object under review (3a) in the viewer's brain is determined (56-60), the brain response comprising a location, frequency and intensity of an EEG signal measured from the viewer's (10) brain, and that the emotional state generated by the object under review (3a) in the viewer (10) is determined from the said brain response.

13. The method according to claim 12, **characterized in that** the viewer's (10) brain response is determined in the data processing means (42) belonging to the 3D virtual environment from the EEG measurement data measured and transmitted by the EEG hat (4) containing several EEG sensors and being located on the viewer's (10) head.

14. The method according to claim 13, **characterized in that** the said data processing means (42) comprises a program application, which edits the EEG measurement data obtained from the EEG sensors of the EEG hat (4) so that it is possible to indicate activations the image of the object under review (3a) shown to the user in the 3D virtual environment cause in different sections of the viewer's (10) brain so that an emotional or cognitive state generated to the viewer (10) of the image under review (3a) is configured to be determined from these activations using neuro analytical methods by comparing the neuro analytical brain response generated by the object under review (3a) to known neuro analytical reference brain responses.

15. The method according to claim 12, **characterized in that** the viewer's (10) location is determined with positioning means (44), which comprise at least one optical camera (44), infrared radar or triangulation equipment operating in a wireless network.

16. The method according to claim 12 or 15, **characterized in that** a viewer's (10) visual target point is determined in the 3D virtual environment by using means for identifying the direction of the eyes.
